(19) Europäisches Patentamt
European Patent Office
Office européen des brevets

(11) EP 4 006 021 A1

(12) EUROPEAN PATENT APPLICATION

(43) Date of publication:
**01.06.2022 Bulletin 2022/22**

(21) Application number: **20210564.9**

(22) Date of filing: **30.11.2020**

(51) International Patent Classification (IPC):
***C07D 333/20*** *(2006.01)* ***H01L 51/00*** *(2006.01)*

(52) Cooperative Patent Classification (CPC):
**C07D 333/20; H01L 51/0059; H01L 51/0068;**
H01L 51/4246

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **TOYOTA JIDOSHA KABUSHIKI KAISHA**
**Aichi-ken, 471-8571 (JP)**

(72) Inventors:
- **KINGE, Sachin**
  **1140 Brussels (BE)**
- **SUTANTO, Albertus Adrian**
  **1950 Sion (CH)**
- **VELLAICHAMY, Joseph**
  **626 111 Meenakshipuram (IN)**
- **CHEN, Ming-Chou**
  **Taiwan (TW)**
- **KHAJA NAZEERUDDIN, Mohammad**
  **1024 Ecublens (CH)**

(74) Representative: **Cabinet Beau de Loménie**
**158, rue de l'Université**
**75340 Paris Cedex 07 (FR)**

# (54) TRIPHENYLAMINE FUNCTIONALIZED OLIGOTHIOPHENES: STABLE AND LOW COST HOLE-TRANSPORTING MATERIALS FOR HIGH PERFORMANCE PEROVSKITE SOLAR CELLS

(57) The present invention relates to a functionalized oligothiophene, represented by the formula (1):

$$A^nB_x \quad (1)$$

wherein $A^n$ is structure consisting of n thiophenes in a non-fused arrangement, wherein n is an integer and x is an integer from 1 to 6, and B is a p-(diaryl-amino)-arylene substituent of formula (2) wherein # represents the point of attachment to the structure $A^n$, and n is an integer:

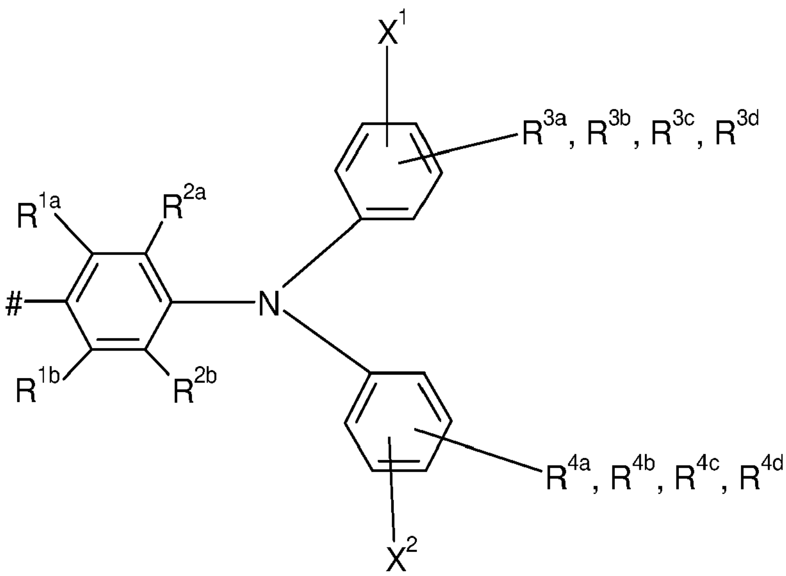

(2)



EP 4 006 021 A1

wherein:
$R^{1a}$, $R^{1b}$, $R^{2a}$, $R^{2b}$, $R^{3a}$, $R^{3b}$, $R^{3c}$, $R^{3d}$, $R^{4a}$, $R^{4b}$, $R^{4c}$, $R^{4d}$ are each independently from one another: a hydrogen atom; a linear or branched alkyl group of 1 to 6 carbon atoms, preferably a methyl or ethyl group; or a halogen atom;
$X^1$ and $X^2$ are each independently from one another: an alkoxy group -O-$R^5$ wherein $R^5$ is a linear or branched alkyl group of 1 to 6 carbon atoms, preferably a methyl or ethyl group;
$X^1$ and $X^2$ are each independently from one another at an ortho, meta or para position with respect to the nitrogen atom N of formula (2);
groups $R^{3a}$, $R^{3b}$, $R^{3c}$ and $R^{3d}$ represent the substituents on the four sites of the aromatic ring bearing group $X^1$ that are neither bound to group $X^1$ nor to the nitrogen atom shown in formula (2), the sites bound to groups $R^{3a}$, $R^{3b}$, $R^{3c}$ and $R^{3d}$ being chosen in any order on the aromatic ring bearing group $X^1$; and
groups $R^{4a}$, $R^{4b}$, $R^{4c}$ and $R^{4d}$ represent the substituents on the four sites of the aromatic ring bearing group $X^2$ that are neither bound to group to group $X^2$ nor to the nitrogen atom shown in formula (2), the sites bound to groups $R^{4a}$, $R^{4b}$, $R^{4c}$ and $R^{4d}$ being chosen in any order on the aromatic ring bearing group $X^2$, and the method of preparation thereof.

The present invention also relates to a perovskite solar cell comprising the functionalized oligothiophene as holle transport material.

## Description

Field of the invention

The present invention relates to functionalized oligothiophenes, their use as low cost, high efficiency and high thermal stability (i.e. at a temperature higher than 400°C) hole transport materials (HTM) in an optoelectronic device, such as solar cells, lasing, light-emitting devices and photodetectors, and optoelectronic devices.

Background art

In the last decade, perovskite solar cells (PSC) have gained a lot of attention due to their high efficiency, up to 25.2% ([1]). The rapid growth in efficiency of PSC emerges as a promising alternative to conventional silicon solar cells.
However, low charge extraction and poor stability of the metal-halide perovskite limit their credentials in large scale applications. In order to overcome these limitations, p-type semi-conducting materials were sandwiched between perovskite and metal electrode which act as hole-transporting materials (HTM). HTMs play a vital role, in PSC, of extracting and transferring the hole charges and in order to achieve high efficiencies ([2], [3], [4]). HTMs can be classified as inorganic polymeric HTMs and small molecular organic HTMs.
Among them, small molecular HTMs are superior to inorganic polymeric HTMs owing to their structural diversity, well defined molecular structure, precise molecular weight and reliable batch-to batch reproducibility. Thus, much research efforts have been devoted to develop small molecular HTMs ([5]). Till now, based on this research, 2,2,7,7-tetrakis(N,N-di-p-methoxyphenylamine)-9,9-spirobifluorene (spiro-OMeTAD) has been widely employed as the benchmark hole transporting material in PSC devices ([5], [6]).
Unfortunately, the complicated synthesis and tedious purification of spiro-OMeTAD makes it expensive. Also, the power conversion efficiency of PSC is highly dependent on the purity of the spiro-OMeTAD employed, which limits its large-scale applications. So, the development of cost-effective hole transporting materials possessing excellent stability, appropriate highest occupied molecular orbital (HOMO) and lowest unoccupied molecular orbital (LUMO) energy levels to transport holes and block electrons respectively, is required for commercialization of perovskite solar cells. Donor-n-acceptor-donor HTMs such as benzene, biphenyl, thiophene, fused thiophenes, anthracene, carbazole, fluorene flanked by arylamine donors have been extensively studied in PSCs to replace expensive spiro-OMeTAD.
The easy synthesis and purification of HTMs have a direct impact on the cost of the device. From the viewpoint of the cost of fabricating HTMs, synthesis must have a lower number of steps and easy purification.
Therefore there is a need for low cost, high efficiency and high thermal stability (at a temperature higher than 400°C) HTMs, which overcome the above-mentioned technical problems.
Oligothiophene-based small molecules are known for their potential applications such as organic thin film transistors, organic light emitting diodes, organic solar cells and perovskite solar cells. Indeed, fused thiophene-based (polymerized) HTMs showed excellent performance in PSCs ([7]-[11]).
The inventors have designed a new structure of non-fused (non-polymerized) thiophene as building blocks to overcome the above-mentioned technical problems.

References:

[1] H.-S. Kim, C.-R. Lee, J.-H. Im, K.-B. Lee, T. Moehl, A. Marchioro, S.-J. Moon, R. Humphry-Baker, J.-H. Yum, J. E. Moser, M. Gratzel and N.-G. Park, et. al. Sci. 2012, Rep. 2, 591;
[2] Y. Chen, Adv. Energy Mater. 2019, 9, 1901268
[3] S. Vegiraju, Adv. Func. Mater. 2019, 1905393
[4] I. Zimmermann, Angew. Chem. Int. Ed 2019, 58, 1072-1076
[5] D. Bi, Sci. Adv. 2016, 2, 1501170
[6] H. Li et al. ChemSusChem 2014, 7, 3420 - 3425
[7] Thirumal et al. J. Mater. Chem. A, 2014, 2, 6305-6309
[8] Mona Shasti et al. Org. Lett. 2019, 21, 3261-3264
[9] Liang Duan, ACS Appl. Energy Mater. 2020, 3, 1672-1683
[10] WO2015114521A1
[11] WO2015161989A1
[12] Macromolecules 1998, 31, 4838-4844
[13] Adv. Funct Mater. 2007, 17, 1163-1171
[14] J. Am. Chem. Soc. 2004, 126, 13480-13501

Summary of the Invention

The present invention relates to a functionalized oligothiophene, represented by the formula (1):

$A^nB_x$ (1)

wherein $A^n$ is structure consisting of n thiophenes in a non-fused arrangement, wherein n is an integer and x is an integer from 1 to 6, and B is a p-(diaryl-amino)-arylene substituent of formula (2) wherein # represents the point of attachment to the structure $A^n$, and n is an integer:

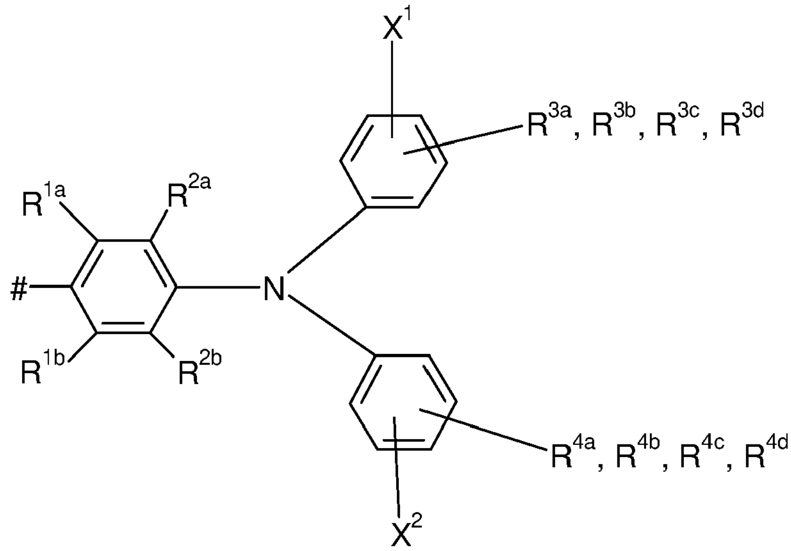

(2)

wherein:

$R^{1a}$, $R^{1b}$, $R^{2a}$, $R^{2b}$, $R^{3a}$, $R^{3b}$, $R^{3c}$, $R^{3d}$, $R^{4a}$, $R^{4b}$, $R^{4c}$, $R^{4d}$ are each independently from one another: a hydrogen atom; a linear or branched alkyl group of 1 to 6 carbon atoms, preferably a methyl or ethyl group; or a halogen atom;
$X^1$ and $X^2$ are each independently from one another: an alkoxy group -O-$R^5$ wherein $R^5$ is a linear or branched alkyl group of 1 to 6 carbon atoms, preferably a methyl or ethyl group;
$X^1$ and $X^2$ are each independently from one another at an ortho, meta or para position with respect to the nitrogen atom N of formula (2);
groups $R^{3a}$, $R^{3b}$, $R^{3c}$ and $R^{3d}$ represent the substituents on the four sites of the aromatic ring bearing group $X^1$ that are neither bound to group $X^1$ nor to the nitrogen atom shown in formula (2), the sites bound to groups $R^{3a}$, $R^{3b}$, $R^{3c}$ and $R^{3d}$ being chosen in any order on the aromatic ring bearing group $X^1$; and
groups $R^{4a}$, $R^{4b}$, $R^{4c}$ and $R^{4d}$ represent the substituents on the four sites of the aromatic ring bearing group $X^2$ that are neither bound to group to group $X^2$ nor to the nitrogen atom shown in formula (2), the sites bound to groups $R^{4a}$, $R^{4b}$, $R^{4c}$ and $R^{4d}$ being chosen in any order on the aromatic ring bearing group $X^2$.

The present invention also relates to a method for preparing the functionalized oligothiophene, comprising the steps of :

(1) Preparing a substituted oligothiophene $A^nL_x$ by reacting n-thiophene, a structure consisting of n thiophenes in a non-fused arrangement wherein x is an integer from 1 to 6, and $L^2$; and
(2) Reacting $A^nL_x$ with a 4-(trialkylstannyl)phenyl-aniline corresponding to formula (2) of claim 1 wherein the element # of formula (2) is replaced by - $Sn(R^5)_3$ wherein each $R^5$ group is, independently of one another, a C1-C6 alkyl group,

wherein L is Br or I, and preferably Br.

The present invention further relates to a perovskite solar cell comprising:

a) a transparent conductive layer;
b) an electron transport layer

c) a perovskite film;
d) a hole transport layer of the functionalized oligothiophene; and
e) an electrode

optionally further comprising one or more of b) an electron transport layer, b') an electron-blocking layer and d') a hole-blocking layer.

Finally the present invention also relates to a use of the functionalized oligothiophene as a hole transport material.

Brief description of the drawings

Figure 1 shows a synthetic route of the functionalized oligothiophenes of the present invention.
Figure 2 shows J-V curves of BT-4D, TT-4D, QT-4D, and spiro-OMeTAD as HTLs.
Figure 3 shows the results of long-term stability test of the unencapsulated devices employing BT-4D and spiro-OMeTAD as HTLs under continuous 1-sun illumination and inert atmosphere for around 1186 h.
Figure 4(a) shows a schematic diagram of PSC device structure. Figures 4(b) shows Cross-sectional scanning electron microscope (SEM) image of the PSC employing BT-4D as HTL. The color code of each layer in the SEM image corresponds to the layers illustrated in (a).
Figure 5 presents thermal properties of the compounds characterized by differential scanning calorimetry (DSC) and thermogravimetric analysis (TGA) recorded under nitrogen atmosphere at the heating rate of 10 °C/min.

Detailed description of the invention

<Functionalized oligothiophene>

One aspect of the present invention relates to a functionalized oligothiophene, represented by the formula (1):

$$A^nB_x \qquad (1)$$

wherein $A^n$ is structure consisting of n thiophenes in a non-fused arrangement, wherein n is an integer and x is an integer from 1 to 6, and B is a p-(diaryl-amino)-arylene substituent of formula (2) wherein # represents the point of attachment to the structure $A^n$, and n is an integer:

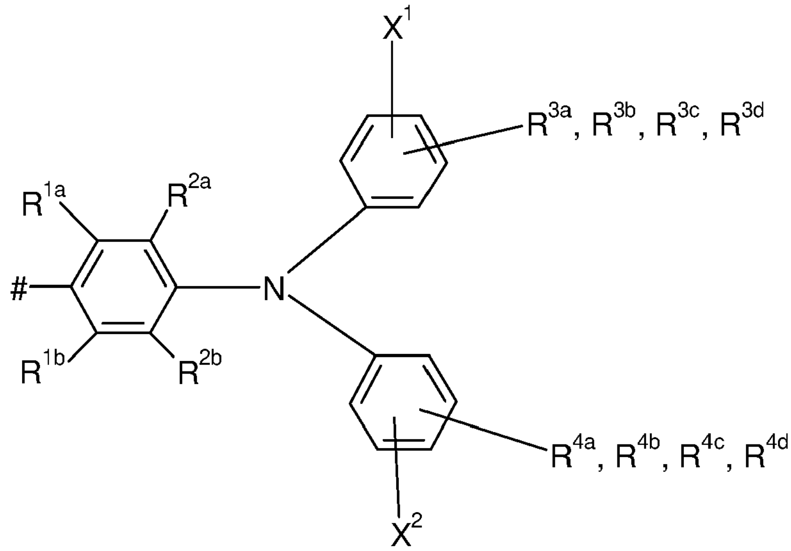

(2)

wherein:

$R^{1a}$, $R^{1b}$, $R^{2a}$, $R^{2b}$, $R^{3a}$, $R^{3b}$, $R^{3c}$, $R^{3d}$, $R^{4a}$, $R^{4b}$, $R^{4c}$, $R^{4d}$ are each independently from one another: a hydrogen atom; a linear or branched alkyl group of 1 to 6 carbon atoms, preferably a methyl or ethyl group; or a halogen atom;

$X^1$ and $X^2$ are each independently from one another: an alkoxy group -O-$R^5$ wherein $R^5$ is a linear or branched alkyl group of 1 to 6 carbon atoms, preferably a methyl or ethyl group;
$X^1$ and $X^2$ are each independently from one another at an ortho, meta or para position with respect to the nitrogen atom N of formula (2);
groups $R^{3a}$, $R^{3b}$, $R^{3c}$ and $R^{3d}$ represent the substituents on the four sites of the aromatic ring bearing group $X^1$ that are neither bound to group $X^1$ nor to the nitrogen atom shown in formula (2), the sites bound to groups $R^{3a}$, $R^{3b}$, $R^{3c}$ and $R^{3d}$ being chosen in any order on the aromatic ring bearing group $X^1$; and
groups $R^{4a}$, $R^{4b}$, $R^{4c}$ and $R^{4d}$ represent the substituents on the four sites of the aromatic ring bearing group $X^2$ that are neither bound to group to group $X^2$ nor to the nitrogen atom shown in formula (2), the sites bound to groups $R^{4a}$, $R^{4b}$, $R^{4c}$ and $R^{4d}$ being chosen in any order on the aromatic ring bearing group $X^2$.

Oligothiophene: $A^n$

Oligothiophene $A^n$ can be a linear or branched oligothiophene, and preferably a linear oligothiophene.
Preferably, n is an integer of 1 or more and 5 or less, and preferably 2 or more and 4 or less.
More preferavbly, $A^n$ is selected from: BT (2,2'-bithiophene), TT (2,2':5',2"-terthiophene) and QT (2,3':2'2":3",2"'-quaterthiophene), each of these oligothiophenes having four binding sites shown by # in the structures below where a thiophene ring is linked covalently to the corresponding # position of formula (2):

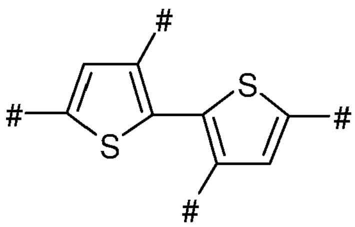

BT

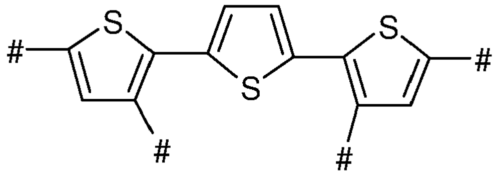

TT

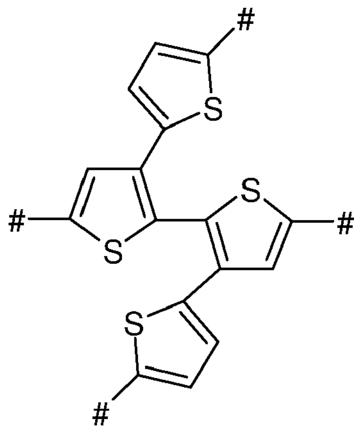

QT

p-(diaryl-amino)-arylene substituent: B

Preferably, all of $R^{1a}$, $R^{1b}$, $R^{2a}$, $R^{2b}$, $R^{3a}$, $R^{3b}$, $R^{3c}$, $R^{3d}$, $R^{4a}$, $R^{4b}$, $R^{4c}$, $R^{4d}$ are hydrogen atoms.
Prefarably, $X^1$ and $X^2$ each represents a methoxy group, and more preferably, $X^1$ and $X^2$ each are at the para-position with respect to the N atom.
More preferably, all of $R^{1a}$, $R^{1b}$, $R^{2a}$, $R^{2b}$, $R^{3a}$, $R^{3b}$, $R^{3c}$, $R^{3d}$, $R^{4a}$, $R^{4b}$, $R^{4c}$, $R^{4d}$ are hydrogen atoms, and each of $X^1$ and $X^2$ is a methoxy or ethoxy group, preferably a methoxy group.

$A^nB_x$

In $A^nB_x$, preferably, x is an integer between 2 and 4, and preferably x is 4, and more preferably, n is 2 or 3 and x is 2 or 4, or n is 4 and x is. Preferably, the other substituent(s) on each thiophene ring, where no B group or link to another thiophene is present, is(are) hydrogen.
Preferably, $A^nB_x$ is represented by the formula:

BT-4D (1)

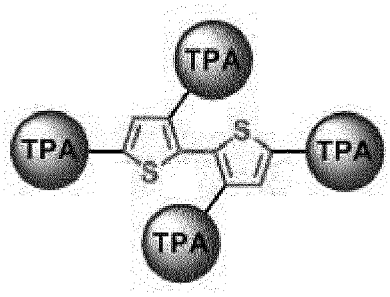

TT-4D (2)

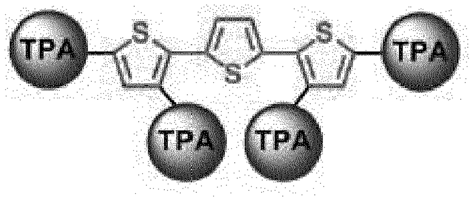

QT-4D (3)

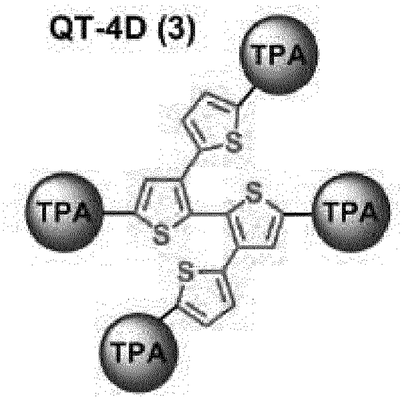

wherein

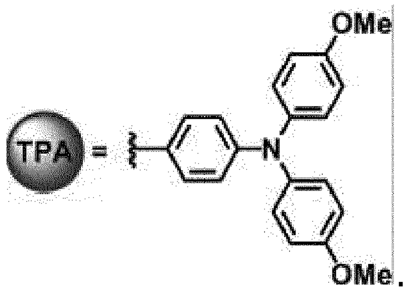

<A method for preparing the functionalized oligothiophene>

Another aspect of the present invention relates to the method of synsthesis of the functionalized oligothiophene.

The method for preparing the functionalized oligothiophene according to any one of claims 1-10, comprising the steps of:

(1) Preparing a brominated oligothiophene $A^nL_x$ by reacting n-thiophene, a structure consisting of n thiophenes in a non-fused arrangement wherein x is an integer from 1 to 6, and $L_2$; and
(2) Reacting $A^nL_x$ with a 4-(trialkylstannyl)phenyl-aniline corresponding to formula (2) of claim 1 wherein the element # of formula (2) is replaced by $-Sn(R^5)_3$ wherein each $R^5$ group is, independently of one another, a C1-C6 alkyl group,

wherein L is Br or I, and preferably Br.

(1) Preparation of a brominated oligothiophene $A^nL_x$

A substituted oligothiophene $A^nL_x$ can be prepared by reacting n-thiophene, a structure consisting of n thiophenes in a non-fused arrangement wherein x is an integer from 1 to 6, and bromine.

Preferably x is 4.

(2) Reacting $A^nL_x$ with a 4-(trialkylstannyl)phenyl-aniline

The 4-(trialkylstannyl)phenyl)aniline used in step (2) is preferably 4-methoxy-N-(4-methoxyphenyl)-N-(4-(tributylstannyl)phenyl)aniline, or 4-methoxy-N-(4-methoxyphenyl)-N-(4-(trimethylstannyl)phenyl)aniline.
In step (2), a palladium (Pd) catalyst can be used in step (2), preferably $Pd(PPh_3)_4$.

<Alternative method of synthesis>

Alternatively, the functionalized oligothiophene of the present invention can be prepared by the following steps:

(1) Preparing bromo or iodo-subtituted oligothiophene;
(2) Preparing a TPA-boronic acid, wherein

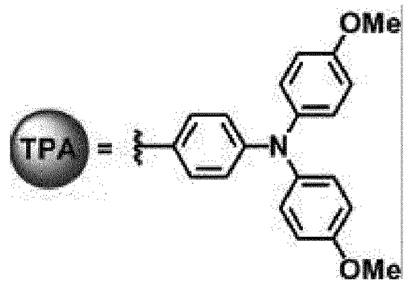

;

(3) Carrying out Suzuki coupling reaction with the bromo or iodo-substituted oligothiophene obtained in step (1) and the TPA-boronic acid obtained in step (2).

<Perovskite solar cell>

Still another aspect of the present invention relate to a perovskite solar cell comprising the functionalized oligothiophene of the present invention as hole transport material.
The perovskite solar cell can comprise the following layers:

a) a transparent conducting layer;
b) an electron transport layer;
c) a perovskite layer;
d) a hole transport layer;
e) an electrode layer

Preferably, a perovskite solar cell can further comprise one or more of: a hole transport layer, an electron transporting layer, an electron-blocking layer and a hole-blocking layer.
The presence of at least one of these layers lead to a higher device efficiency.
The above-mentioned layers can be disposed in this order. An electron transport layer can be situated between the transparent conducting layer and the perovskite layer and a hole transport layer can be situated between the perovskite layer and an electrode layer.
In one embodiment, the perovskite solar cell may have a conventional n-i-p structure comprising a transparent conducting layer, an electron transport later, a perovskite layer, a hole transport layer and an electrode layer in this order.
In another embodiment, the perovskite solar cell may have an inverted p-i-n structure comprising a transparent conducting layer, a hole transport later, a perovskite layer, an electron transport layer and an electrode layer in this order.
In still another embodiment, the perovskite solar cell can further comprise mesoporous scaffold, in the perovskite layer.
Generally, in the so-called p-i-n or n-i-p structures, the electron (n) or hole (p) blocking layers are situated in the bottom and top of the perovskite layer (i), in a sandwich configuration, with the perovskite layer (i) being in the middle. A mesoporous $TiO_2$ layer is usually an electron transport layer (in the n side).

<a) Transparent conductive layer>

A transparent conductive layer is not particularly limited and can comprise or consist of, for example, a fluorine-doped tin oxide (FTO), indium tin oxide (ITO), doped zinc oxide, carbon nanotube networks or graphene, and preferably a FTO.

<b) Electron transport layer (ETL)>

The electron transport layer is not particularly limited and can comprise or consist of, for example, $TiO_2$, $SnO_2$, Nb-doped $SnO_2$, Sb-doped $SnO_2$, C60 and C60 derivatives, bathocuproine (BCP), a combination of C60/BCP, and a combination of $TiO_2/SnO_2$ bilayer.

Among these materials, a combination of $TiO_2/SnO_2$ layer is preferred for n-i-p configuration, and a combination of C60/BCP is preferred for p-i-n configuration.

<b') Hole-blocking layer>

An optional hole blocking layer can be, for example, 2,9-dimethyl-4, 7-diphenylphenanthroline (BCP), $TiO_2$, $SnO_2$, ZnO.

When the electron transport layer (ETL) material is $TiO_2$, $TiO_2$ behaves as both electron transport and hole blocking, given that the layer is compact. However if the ETL material is mesoporous, the perovskite solution infiltrates and touches the FTO electrode (which will produce losses). In order to avoid the direct contact between perovskite and FTO when the ETL material is mesoporous, normally a compact layer of $TiO_2$ is incorporated between FTO and mesoporous-$TiO_2$ (blocking layer).

<c) Perovskite layer>

The perovskite layer/film can be formed, for example, by spin-coating, printing, slot-die coating and meniscus coating. The slot-die coating method is preferable for large-area deposition.

The temperature for the crystallization can be, for example, around 100°C.

The fabrication of perovskite film can be, for example, carried inside the $N_2$ glovebox with the moisture value of <0.1 ppm, or under the ambient air.

Perovskite precursors

The at least one organic halide compound in the (A) precursor can be, for example, at least one selected from the group consisting of methylammonium (MA), formamidinium (FA), phenylethylammonium (PEA) and guanidinium (GUA). Particular examples of metal halide in the (B) precursors are $PbCl_2$, $PbBr_2$, $PbI_2$. It may be noted that metal carboxylates, particularly lead (Pb) carboxylates such as Pb $(CH_3COO)_2$ may be used instead of metal halides.

Perovskite coating solution

The solvent is not particularly limited and can be, for example, dimethylsulfoxide (DMSO), dimethylformamide (DMF), a combination of DMSO:DMF, gamma-butyrolactone (GBL), N-methyl-2-pyrrolidone (NMP), dimethylacetamide (DMAc) and acetonitrile (ACN).

A preferred solvent is, for example, DMSO or a combination of DMSO:DMF.

<d') Electron-blocking layer>

An optional electron blocking layer can be, for example, AIGaN.

<d) Hole transport layer>

The hole transport layer is made of the functionalized oligothiophene of the present invention.

<e) Electrode layer>

The electrode layer is not particularly limited and can comprise or consist of, for example, Au, C, Ag, Cu or Al. Among these, Au is preferred in the laboratory scale since it is very efficient and stable but expensive. For industrial applications, carbon and Cu are preferred.

<Method of preparing a solar cell device comprising a perovskite film>

The present invention also relates to a method of preparing a solar cell device comprising a perovskite film. The method of the present invention comprises the following steps:

1) preparing a transparent conducting oxide layer;
2) optionally preparing and depositing an electron transport layer;
3) optionally preparing and depositing a hole-blocking layer;
4) preparing and depositing a perovskite layer;
5) optionally preparing and depositing an electron-blocking layer
6) optionally preparing and depositing a hole transport layer; and
7) preparing and depositing an electrode layer

1) Preparing a conducting oxide layer

A conductive oxide layer can be prepared on an FTO glass. An etched FTO glass is cleaned and then its surface is treated, and can be later treated for example by spin-coating a $TiO_2$ layer which acts as electron transport layer.
A person skilled in the art knows applying an appropriate preparation method for a given conducting oxide layer material. Commercial FTO-glass substrates are available, and can be used in the present invention, wherein a conductive FTO layer has already been deposited on top of a glass substrate. Etching may then be carried out to pattern the FTO followed by surface cleaning.

2) Optionally preparing and depositing an electron transport layer (ETL)

An optional electron transport layer can be prepared with $TiO_2$, for example by spin-coating followed by annealing.
When the ETL material is $TiO_2$, $TiO_2$ behaves as both electron transport and hole blocking material, given that the layer is compact. However if the ETL material is mesoporous, the perovskite solution infiltrates and touches the FTO electrode (which will produce losses). In order to avoid the direct contact between perovskite and FTO when the ETL material is mesoporous, normally a compact layer of $TiO_2$ is incorporated between FTO and mesoporous-$TiO_2$ (blocking layer). Therefore optionally, a $TiO_2$-blocking layer can be deposited before between the conductive layer and an electron transport layer.

3) Optionally preparing and depositing a hole-blocking layer

A person skilled in the art knows applying an appropriate preparation method for a hole-blocking material.
If the hole-blocking layer material is an organic material, line BCP, it can be prepared from solution (spin coating from organic solvent like chlorobenzene) or via thermal sublimation.
If the hole-blocking layer material is an inorganic material, like $TiO_2$, it can be prepared by spin-coating a precursor solution and then thermal annealing. For example, a diluted titanium diisopropoxide bis(acetylacetonate) solution (Sigma-Aldrich) in ethanol by spray pyrolysis at 450C.
A person skilled in the art knows applying an appropriate preparation method for a given electron transport layer material.

4) Preparing and depositing a perovskite layer

A perovskite solar layer can be deposited by the method as disclosed above, using a perovskite precursor solution as disclosed above. This step can be conducted in ambient air (the temperature was around 25 °C and humidity was about 30-50% RH).
A person skilled in the art knows applying an appropriate preparation method for a given perovskite layer material.

5) Optionally preparing and depositing an electron-blocking layer

A person skilled in the art knows applying an appropriate preparation method for an electron-blocking material.
If the electron-blocking layer material is an organic material it could be added from solution (spin coating), like for Spiro-OMeTAD, PEDOT:PSS, PolyTPD, PTAA.
If the electron-blocking layer material is NiOx, it can be prepared from spin-coating a precursor solution and then thermal annealing.

6) Preparing and depositing a hole transport layer (HTL)

The functionalized oligothiophene of the present invention is deposited as HTL. An optional hole transport layer can then be formed for example by spin-coating. This step can be conducted in ambient air (the temperature was around 25 °C and humidity was about 30-50% RH).
A person skilled in the art knows applying an appropriate preparation method for a given hole transport layer material.

7) Preparing and depositing an electrode layer

An electrode layer can be prepared for example by evaporating Au.
A person skilled in the art knows applying an appropriate preparation method for an electrode layer material.
In the method of preparing a solar cell according to the invention, the method may appropriately be carried out under a relative humidity of 10% or more and 60% or less, preferably 20% or more and 50% or less, more preferably 25% or more and 45% or less, and 3 most preferably 0% or more and 40% or less.
In the method of preparing a solar cell according to the invention, in some embodiments, at least one of the steps (1) and (3) may be carried out under an ambient temperature of 15 °C or more and 30 °C or less, preferably 15°C or more and 25°C or less and most preferably 20°C or more and 25°C or less. However, the temperature of the steps may vary. The perovskite-containing layer may be appropriately deposited in ambient temperature but then annealed at a temperature of around 100°C. The steps (1) and (3) can be or not, depending on the material, carried out at ambient temperature, but for example the common $TiO_2$ electron transport material is deposited in ambient temperature and then annealed at high temperature 500 °C. There are alternatives, such as $SnO_2$ deposited at 150°C.
In the present invention, triarylamine substituted oligothiophenes have been designed and synthesized as small molecular hole transporting materials for perovskite solar cells. The device fabricated with triphenylamine substituted bithiophene (BT-4D) showed the power conversion efficiency (PCE) of 19.25% which is the best efficiency among the devices fabricated with thiophene, bithiophene-based small molecular hole transporting materials. It has been demonstrated that non-fused oligothiophenes are a promising building block for achieving highly efficient and cost-effective perovskite solar cells.
The present HTMs are useful, in particular in solid-state solar cells, including perovskite/perovskite, silicon/perovskite, and CIGS/perovskite tandem solar cells. The present HTMs have a remarkable stability, where the decrease in the initial efficiency after 1186 hours light exposure is less than 2%.

## Examples

The present invention is detailed below with examples, but the scope of the present invention is not to be considered to be limited to the following examples.

### Synthesis

The synthetic route for the target compounds is drawn in Figure 1. The intermediates 3,3',5,5'-tetrabromo-2,2'-bithiophene, 3,3",5,5"-tetrabromo-2,2':5',2"-terthiophene and 5,5',5",5'"-tetrabromo-2,3':2',2":3",2'"-quaterthiophene were synthesized as indicated below, according to the method disclosed in above-cited references [12], [13] and [14].

**Synthesis of 3,3',5,5'-tetrabromo-2,2'-bithiophene:**

To a solution of 2,2'-bithiophene (0.2 g, 1.2 mmol) in chloroform (10 mL)/glacial acetic acid (2 mL), bromine (0.31 mL, 6 mmol) dissolved in 5 mL of chloroform was added at room temperature. Then it was refluxed for 12 h. After 12 h, it was cooled to room temperature. 20 mL of a saturated aqueous solution of $NaHSO_3$ was added. After extraction with DCM, the organic phase was washed with saturated aqueous $NaHCO_3$ and NaCl and dried over $Na_2SO_4$. After solvent evaporation, the white color solid was obtained.

**Synthesis of 3,3",5,5"-tetrabromo-2,2':5',2"-terthiophene:**

To a solution of 2,2':5',2"-terthiophene (0.2 g, 0.81 mmol) in chloroform (10 mL), bromine (0.17 mL, 3.32 mmol) dissolved in 5 mL of chloroform was added dropwise. The reaction mixture was allowed to stir at room temperature for 12 h. 20 mL of a saturated aqueous solution of $NaHSO_3$ was added. After extraction with DCM, the organic phase was washed with saturated aqueous $NaHCO_3$ and NaCl and dried over $Na_2SO_4$. After solvent evaporation, the yellow color solid was obtained.

**Synthesis of tetrabromo-3,3-dithienyl-2,2-bithiophene:**

To a solution of 3,3-di-(2-thienyl)-2,2-bithiophene (0.2 g in 30 mL of chloroform) was added dropwise a solution of 0.13 mL of $Br_2$ (4.2 equiv) in 10 mL of chloroform. After 15 min stirring, 20 mL of a saturated aqueous solution of $NaHSO_3$ were added. After extraction with DCM, the organic phase was washed with saturated aqueous $NaHCO_3$ and NaCl and dried over $Na_2SO_4$. After solvent evaporation, the residue was recrystallized in a mixture of 5 mL of ethanol and 4 mL of chloroform to give a white solid.

**Synthesis of BT-4D (1)**

A mixture of 3,3',5,5'-tetrabromo-2,2'-bithiophene (0.1 g, 0.21 mmol), 4-methoxy-*N*-(4-methoxyphenyl)-*N*-(4-(tributylstannyl)phenyl)aniline (0.55 g, 0.92 mmol), $Pd(PPh_3)_4$ (97 mg, 0.084 mmol) and toluene (30 mL) was refluxed under nitrogen atmosphere for 24 h. After completion of reaction, the reaction mixture was allowed to room temperature and quenched by addition of water. The organic compound was extracted using dichloromethane and washed with brine solution. The combined organic layer was dried over sodium sulfate and concentrated to get crude sample which then purified by column chromatography. Eluent: Hexanes/Ethyl acetate (3:2). Yellow solid. Yield 0.14 g (48%).

**Synthesis of TT-4D (2)**

It was prepared from a mixture of 3,3",5,5"-tetrabromo-2,2':5',2"-terthiophene (0.1 g, 0.18 mmol), 4-methoxy-*N*-(4-methoxyphenyl)-*N*-(4-(tributylstannyl)phenyl)aniline (0.53 g, 0.90 mmol), $Pd(PPh_3)_4$ (83 mg, 0.07 mmol) and toluene (30 mL) by following the procedure described for BT-4D. Eluent: Hexanes/Ethyl acetate (3:2). Red solid. Yield 0.14 g (54%).

**Synthesis of QT-4D (3)**

It was prepared from a mixture of 5,5',5",5"'-tetrabromo-2,3':2',2":3",2"'-quaterthiophene (0.1 g, 0.15 mmol), 4-methoxy-*N*-(4-methoxyphenyl)-*N*-(4-(tributylstannyl)phenyl)aniline (0.39 g, 0.66 mmol), $Pd(PPh_3)_4$ (69 mg, 0.06 mmol) and toluene (30 mL) by following the procedure described for BT-4D. Eluent: Hexanes/Ethyl acetate (3:2). Yellow solid. Yield 0.12 g (52%).

The metal-catalyzed Stille coupling reactions of 4-methoxy-N-(4-methoxyphenyl)-N-(4-(tributylstannyl)phenyl)aniline with the bromo intermediates under nitrogen atmosphere yielded the target molecules. The crude product is purified by column chromatography technique by using hexanes/ethyl acetate (3:2) as eluent. The obtained compounds (BT-4D and QT-4D) are yellow in color while TT-4D red in color. All the compounds are found to be soluble in common organic solvents which make them facile for solution processed device fabrication.

The functionalized oligothiophenes thus synthesized were then purified by column chromatography using hexanes/ethyl acetate (3:2) as eluent.

The obtained products were thoroughly characterized by $^1H$ NMR, $^{13}C$ NMR spectroscopy and HRMS analysis and the obtained results were found to be consistent with the proposed molecular structure.

Thermal property

Thermal properties of the compounds were characterized by differential scanning calorimetry (DSC) and thermogravimetric analysis (TGA) recorded under nitrogen atmosphere at the heating rate of 10 °C/min. All the compounds showed high thermal decomposition temperature of greater than 400 °C corresponding to 5% weight loss (Figure 5). The high decomposition temperature of the compounds supports the long durability of the devices.

Table 1: Thermal, optical and electrochemical properties of BT-4D, TT-4D and QT-4D

| Compound | $T_d$ [°C] | $T_m$ [°C] | $\lambda_{abs}$ (soln) [nm] | $E_{ox}$ [V] | HOMO [eV] | LUMO [eV] | $\Delta_{Eg}$ [eV]g | $\lambda_{onset}$ (soln) [nm] |
|---|---|---|---|---|---|---|---|---|
| BT-4D | 403 | 190 | 366 | 0.74 | -5.22 | -2.66 | 2.56 | 485 |
| TT-4D | 421 | 125 | 443 | 0.75 | -5.23 | -2.92 | 2.31 | 537 |

(continued)

| Compound | $T_d$ [°C] | $T_m$ [°C] | $\lambda_{abs}$ (soln) [nm] | $E_{ox}$ [V] | HOMO [eV] | LUMO [eV] | $\Delta_{Eg}$ [eV]g | $\lambda_{onset}$ (soln) [nm] |
|---|---|---|---|---|---|---|---|---|
| QT-4D | 427 | 182 | 388 | 0.82 | -5.30 | -2.66 | 2.64 | 470 |

$T_d$: Decomposition temperature corresponding to 5% weight loss determined from TGA

$T_m$: Melting temperature

$\lambda_{abs}$ (soln); Absorption spectra measured in o-dichlorobenezene

$E_{ox}$: Determined from DPV in o-dichlorobenzene at 25 °C with reference to $F_c/F_c^+$ internal standard (at +0.6 V)

$$HOMO = - -(4.44+0.64+E_{ox})$$

$$LUMO = HOMO + \Delta_{Eg}$$

$$\Delta_{Eg} = 1240/\lambda_{onset}$$

$\lambda_{onset}$ = Wavelength at 5% absorption intensity.

Device fabrication

➢ PSC device comprising the functionalized oligothiophene of the present invention as HTM

A PSC device was prepared as shown in Figure 4.

< Preparing a transparent conductive layer>

Fluorine-doped tin oxide (FTO, Nippon Sheet Glass, TEC-9) substrate was patterned by laser etching and cleaned with detergent (Helmanex), deionized water, acetone, and isopropanol (IPA) in an ultrasonic bath for 15 min in each solvent.

<Preparing a compact $TiO_2$ (c-$TiO_2$)-blocking layer>

A $TiO_2$-blocking layer was prepared by by spraying titanium diisopropoxide bis(acetylacetonate) solution (Sigma Aldrich) diluted in isopropanol (1:15 v/v) at 450 °C.

<Preparing a mesoporous $TiO_2$ scaffold layer>

A mesoporous $TiO_2$ layer was prepared by spin-coating $TiO_2$ paste (GreatCellSolar, 30NR-D) in ethanol (1:8 w/v) at 5000 rpm for 20 s. The layer was then heated at 100 °C for 10 min, followed by thermal sintering at 500 °C for 20 min.

< Preparing a $SnO_2$ layer >

A $SnO_2$ layer was prepared by spin-coating aqueous $SnCl_4$ solution (Acros, 12 μL in 988 μL water) at 3000 rpm for 30 s and then heated at 100 °C for 10 min followed by annealing at 190 °C for 1 h.

<Preparing and depositing a perovskite layer>

The perovskite layer was prepared by spin-coating 1.3 M perovskite solution with the composition of $[(FAPbI_3)_{0.87}(MAPbBr_3)_{0.13}]_{0.92}(CsPbI_3)_{0.08}$ which was prepared by mixing the corresponding salts (FAI (GreatCellSolar), MABr (GreatCellSolar), CsI (ABCR), $PbI_2$ (TCI), and $PbBr_2$ (TCI)) in DMF and DMSO (0.78:0.22 v/v). A 5% excess $PbI_2$ ($PbI_2$:FAI = 1.05:1) was used in the precursor solution. To deposit the perovskite layer, the prepared perovskite solution was spin-coated at 2000 rpm for 12 s and 5000 rpm for 30 s. Ethyl acetate was used as an anti-solvent and dropped at 15 s before the spin coating finished. The layer was then annealed at 100°C for 60 minutes.

<Preparing and depositing a hole transport layer>

The hole transport material (HTM) solution was deposited by spin-coating at 4000 rpm for 30s. For the thiophene-based HTMs (BT-4D, TT-4D, and QT-4D), a 36 mM solution was prepared in chlorobenzene and doped with Li-TFSI:tBP:FK209 = 0.5:3.3:0.03 (mol/mol HTM).

<Preparing and depositing an electrode layer>

Finally, the Au electrode was evaporated onto the HTM layer with a thickness of 70 nm.

➢ PSC device comprising spiro-OMeTAD as HTM

PSC devices comprising spiro-OMeTAD as the HTL were also fabricated in the same device configuration as the control device. The HTLs were prepared using solution process and the solutions were doped with Li-bis(trifluoromethanesulphonyl) imide (Li-TFSI), tris(2-(1H-pyrazol-1-yl)-4-tert-butylpyridine)cobalt(III) (FK-209), and 4-tert-butylpyridine (tBP). A 60 mM solution of 2,2',7,7'-tetrakis-(N,N-di-p-methoxyphenyl-amine)-9,9'-spirobifluorene (spiro-OMeTAD, Merck) was prepared in chlorobenzene and doped using Li-bis (trifluoromethanesulphonyl) imide (Li-TFSI, Aldrich), FK-209 Co(III) TFSI (GreatCellSolar), and 4-tert-butylpyridine (tBP, Sigma-Aldrich) with the ratio of Li-TFSI:tBP:FK209 = 0.5:3.3:0.055 (mol/mol spiro-OMeTAD). The prepared solution was then deposited by spin-coating at 4000 rpm for 30s.

Scanning electron microscope (SEM) analyses

Scanning electron microscope (SEM) analyses were performed on the complete solar cell devices to further know how the HTLs is configured on top of perovskite. The cross-sectional SEM images confirm that the deposition of BT-4D, TT-4D, and QT-4D layers result in the formation of about 150 nm-thick HTLs, while the deposition of spiro-OMeTAD results in the formation of about 250 nm-thick HTL (see Figure 4). It can be observed that PSCs samples exhibit dense and uniform layers on top of perovskite.

These conclusions are consistent with photovoltaic data shown in Figure 2.

Stability test of perovskite solar cells

The stability tests of perovskite solar cells with functionalized oligothiophene HTL were carried out on the unencapsulated devices under continuous 1 sun illumination and maximum power point tracking (MPPT) in an inert atmosphere for 1186 hours. The long-term stability test results are reported in Figure 3.

During the first 15 hours, the device based on BT-4D shows a slight increase of 4% compared to the initial PCE, followed by a slow degradation process, retaining 98% of its initial PCE after 1186 hours of light exposure. On the contrary, the device based on state-of-the-art HTL spiro-OMeTAD exhibits a fast degradation behavior on the first 50 hours followed by a slow degradation process, retaining 79% of its initial PCE.

These results prove the superior stability of BT-4D under continuous light illumination due to coordination to Pb2+, outperforming spiro-OMeTAD, with a negligible loss of 2% from the initial PCE after 1186 hours. This outstanding stability of BT-4D makes BT-4D one of the most stable doped HTLs among the other reported doped HTLs with similar device photovoltaic performance to spiro-OMeTAD.

## Claims

1. A functionalized oligothiophene, represented by the formula (1):

$$A^nB_x \qquad (1)$$

wherein $A^n$ is structure consisting of n thiophenes in a non-fused arrangement, wherein n is an integer and x is an integer from 1 to 6, and B is a p-(diaryl-amino)-arylene substituent of formula (2) wherein # represents the point of attachment to the structure $A^n$, and n is an integer:

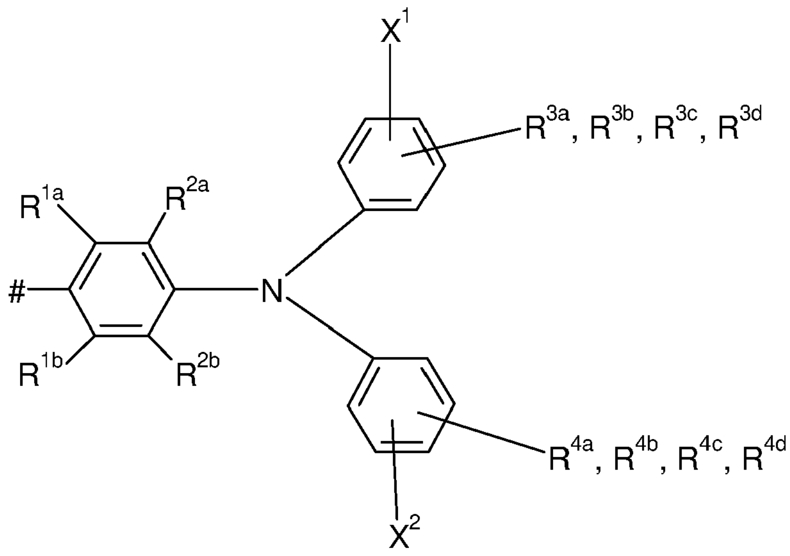

(2)

wherein:

$R^{1a}$, $R^{1b}$, $R^{2a}$, $R^{2b}$, $R^{3a}$, $R^{3b}$, $R^{3c}$, $R^{3d}$, $R^{4a}$, $R^{4b}$, $R^{4c}$, $R^{4d}$ are each independently from one another: a hydrogen atom; a linear or branched alkyl group of 1 to 6 carbon atoms, preferably a methyl or ethyl group; or a halogen atom;
$X^1$ and $X^2$ are each independently from one another: an alkoxy group -O-$R^5$ wherein $R^5$ is a linear or branched alkyl group of 1 to 6 carbon atoms, preferably a methyl or ethyl group;
$X^1$ and $X^2$ are each independently from one another at an ortho, meta or para position with respect to the nitrogen atom N of formula (2);
groups $R^{3a}$, $R^{3b}$, $R^{3c}$ and $R^{3d}$ represent the substituents on the four sites of the aromatic ring bearing group $X^1$ that are neither bound to group $X^1$ nor to the nitrogen atom shown in formula (2), the sites bound to groups $R^{3a}$, $R^{3b}$, $R^{3c}$ and $R^{3d}$ being chosen in any order on the aromatic ring bearing group $X^1$; and
groups $R^{4a}$, $R^{4b}$, $R^{4c}$ and $R^{4d}$ represent the substituents on the four sites of the aromatic ring bearing group $X^2$ that are neither bound to group to group $X^2$ nor to the nitrogen atom shown in formula (2), the sites bound to groups $R^{4a}$, $R^{4b}$, $R^{4c}$ and $R^{4d}$ being chosen in any order on the aromatic ring bearing group $X^2$.

**2.** The functionalized oligothiophene according to claim 1, wherein $A^n$ is a linear or branched oligothiophene, and preferably a linear oligothiophene.

**3.** The functionalized oligothiophene according to claim 1 or 2, wherein n is an integer of 1 or more and 5 or less, and preferably 2 or more and 4 or less.

**4.** The functionalized oligothiophene according to any one of claims 1 to 3, wherein $A^n$ is selected from: BT (2,2'-bithiophene), TT (2,2':5',2"-terthiophene) and QT (2,3':2'2":3",2"'-quaterthiophene), each of these oligothiophenes having four binding sites shown by # in the structures below where a thiophene ring is linked covalently to the corresponding # position of formula (2):

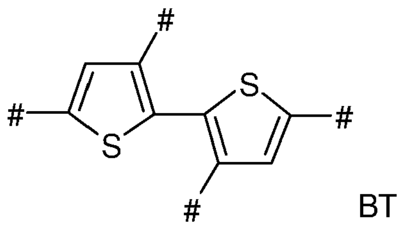

BT

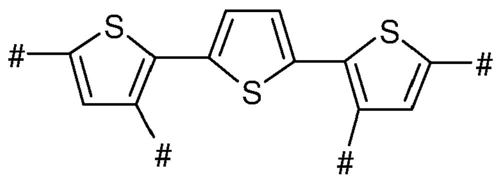

TT

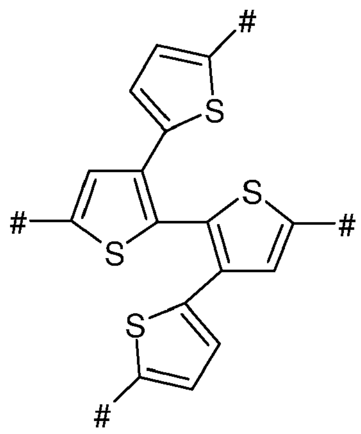

QT

5. The functionalized oligothiophene according to any one of claims 1 to 4, wherein all of $R^{1a}$, $R^{1b}$, $R^{2a}$, $R^{2b}$, $R^{3a}$, $R^{3b}$, $R^{3c}$, $R^{3d}$, $R^{4a}$, $R^{4b}$, $R^{4c}$, $R^{4d}$ are hydrogen atoms.

6. The functionalized oligothiophene according to any one of claims 1 to 5, wherein $X^1$ and $X^2$ each represents a methoxy group.

7. The functionalized oligothiophene according to any one of claims 1 to 6, wherein $X^1$ and $X^2$ each are at the para-position with respect to the N atom.

8. The functionalized oligothiophene according to any one of claims 1 to 7, wherein in group B, all of $R^{1a}$, $R^{1b}$, $R^{2a}$, $R^{2b}$, $R^{3a}$, $R^{3b}$, $R^{3c}$, $R^{3d}$, $R^{4a}$, $R^{4b}$, $R^{4c}$, $R^{4d}$ are hydrogen atoms, and each of $X^1$ and $X^2$ is a methoxy or ethoxy group, preferably a methoxy group.

9. The functionalized oligothiophene according to any one of claims 1 to 8, wherein x is an integer between 2 and 4, and preferably x is 4.

10. The functionalized oligothiophene according to any one of claims 1 to 9, wherein n is 2 or 3 and x is 2 or 4.

11. The functionalized oligothiophene according to any one of claims 1 to 9, wherein n is 4 and x is 4.

12. The functionalized oligothiophene according to any one of claims 1 to 11, represented by the formula:

BT-4D (1)

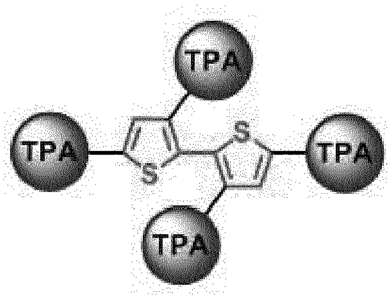

TT-4D (2)

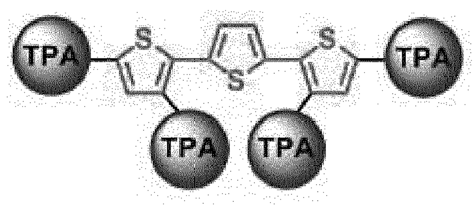

QT-4D (3)

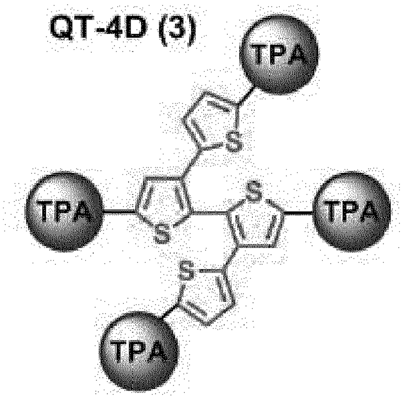

wherein

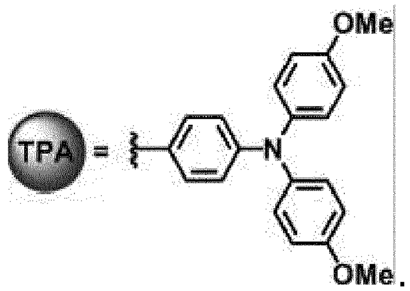

13. A method for preparing the functionalized oligothiophene according to any one of claims 1-12, comprising the steps of:

(1) Preparing a substituted oligothiophene $A^nL_x$ by reacting n-thiophene, a structure consisting of n thiophenes in a non-fused arrangement wherein x is an integer from 1 to 6, and $L_2$; and
(2) Reacting $A^nL_x$ with a 4-(trialkylstannyl)phenyl-aniline corresponding to formula (2) of claim 1 wherein the element # of formula (2) is replaced by $-Sn(R^5)_3$ wherein each $R^5$ group is, independently of one another, a C1-C6 alkyl group,

wherein L is Br or I, and preferably Br.

14. The method according to claim 13 wherein the 4-(trialkylstannyl)phenyl)aniline used in step (2) is 4-methoxy-N-(4-methoxyphenyl)-N-(4-(tributylstannyl)phenyl)aniline, or 4-methoxy-N-(4-methoxyphenyl)-N-(4-(trimethylstannyl)phenyl)aniline.

15. The method according to claim 13 or 14 wherein x is 4.

16. The method according to claims 13 to 15 wherein a palladium (Pd) catalyst is used in step (2), preferably $Pd(PPh_3)_4$.

17. A perovskite solar cell

a) a transparent conductive layer;
b) an electron transport layer
c) a perovskite film;
d) a hole transport layer of the functionalized oligothiophene according to any one of claims 1-12; and
e) an electrode

optionally further comprising one or more of b) an electron transport layer, b') an electron-blocking layer and d') a hole-blocking layer.

18. The perovskite solar cell according to claim 17 wherein a) is a fluorine-doped tin oxide (FTO), indium tin oxide (ITO), doped zinc oxide, carbon nanotube networks or graphene, and preferably a FTO.

**19.** The perovskite solar cell according to claim 17 or 18 wherein b) is $TiO_2$, $SnO_2$, Nb-doped $SnO_2$, Sb-doped $SnO_2$, C60 and C60 derivatives, bathocuprione (BCP), a combination of C60/BCP, a combination of $TiO_2/SnO_2$ bilayer, and preferably $TiO_2/SnO_2$ or a combination of C60/BCP.

**20.** The perovskite solar cell according to any one of claims 17 to 19, wherein e) is Au, C, Ag, Cu or Al, and preferably Au or Cu.

**21.** The perovskite solar cell according to any one of claims 17 to 20, wherein the solar cell further comprises mesoporous scaffold, either in n-i-p or p-i-n configuration.

**22.** Use of the functional oligothiophene according to any one of claims 1 to 12, as hole transport material.

**23.** Use according to claim 22, in an optoelectronic device, such as solar cells, lasing, light-emitting devices and photodetectors.

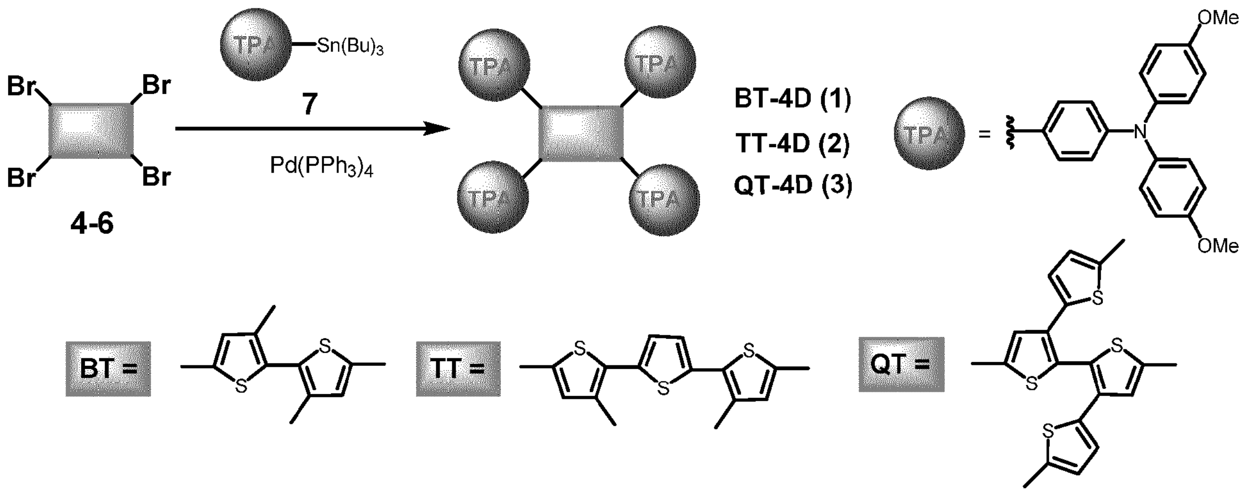


4-6

7

BT-4D (1)
TT-4D (2)
QT-4D (3)

BT =

TT =

QT =

**Figure 1**

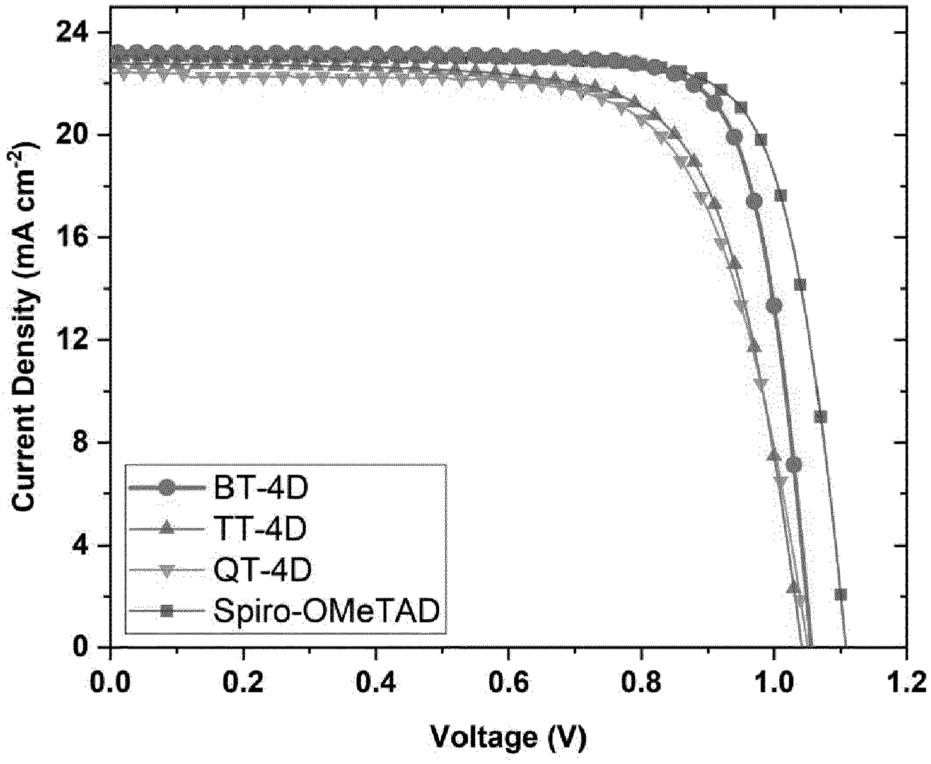

Current Density (mA cm$^{-2}$)
Voltage (V)
BT-4D
TT-4D
QT-4D
Spiro-OMeTAD
0
4
8
12
16
20
24
0.0
0.2
0.4
0.6
0.8
1.0
1.2


**Figure 2**

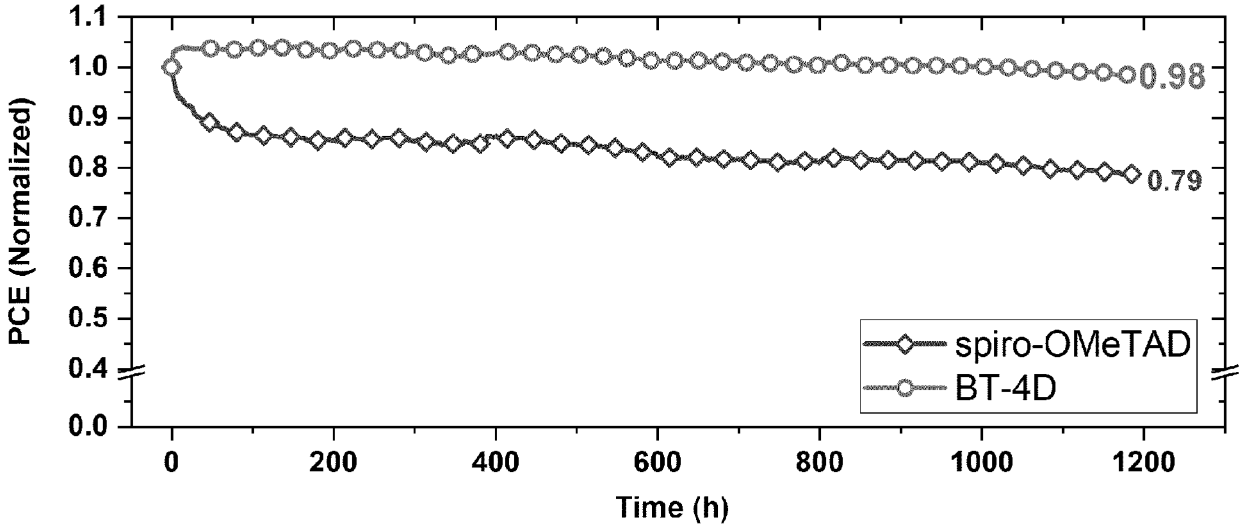
1.1
1.0
0.9
0.8
0.7
0.6
0.5
0.4
0.0
PCE (Normalized)
0
200
400
600
800
1000
1200
Time (h)
0.98
0.79
spiro-OMeTAD
BT-4D

**Figure 3**

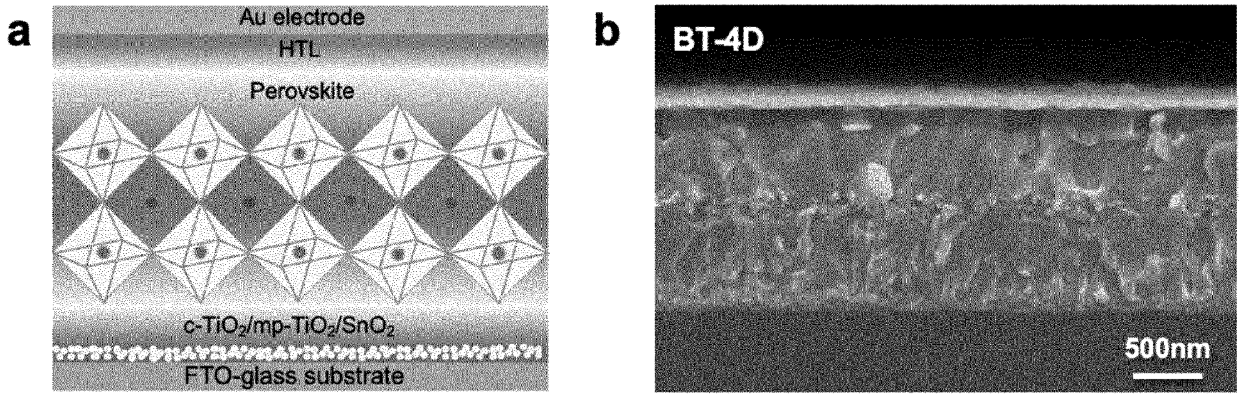
a
Au electrode
HTL
Perovskite
c-TiO2/mp-TiO2/SnO2
FTO-glass substrate
b
BT-4D
500nm

**Figure 4**

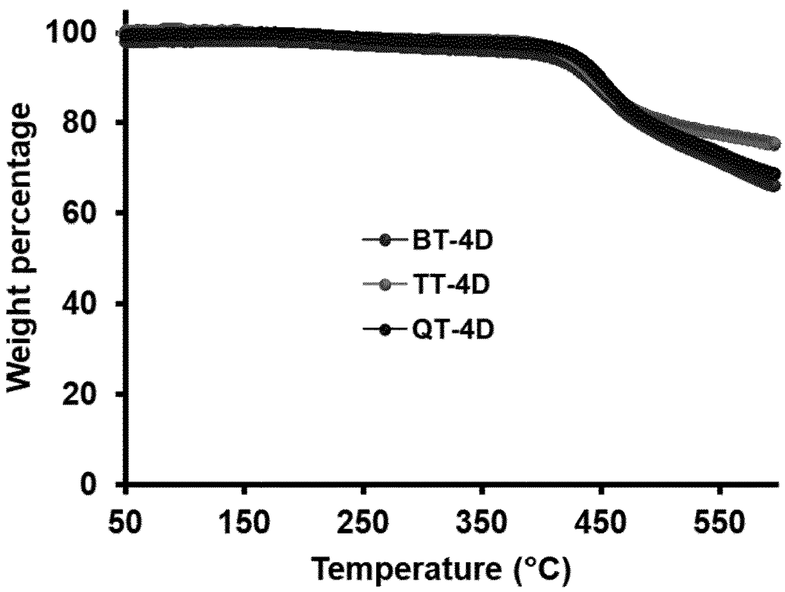
100
80
60
40
20
0
Weight percentage
BT-4D
TT-4D
QT-4D
50
150
250
350
450
550
Temperature (°C)

**Figure 5**

Europäisches Patentamt
European Patent Office
Office européen des brevets

# EUROPEAN SEARCH REPORT

Application Number
EP 20 21 0564

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/084256 A1 (UNIV NANYANG TECH [SG]; DYESOL LTD [AU]) 11 June 2015 (2015-06-11)<br>* claims 1, 27, 55 *<br>* page 12, paragraph 57 *<br>* page 31; compound H102 *<br>* page 21; compounds H111, H112 *<br>* figures 1, 10 *<br>* page 21, paragraph 111 *<br>* page 32, paragraph 153 *<br>----- | 1-3,5-9, 13-23 | INV.<br>C07D333/20<br>H01L51/00 |
| X | BRIGITTE HOLZER ET AL: "Color Fine-Tuning of Optical Materials Through Rational Design",<br>CHEMPHYSCHEM - A EUROPEAN JOURNAL OF CHEMICAL PHYSICS & PHYSICALCHEMISTRY.,<br>vol. 18, no. 5,<br>2 February 2017 (2017-02-02), pages 549-563, XP055463991,<br>DE<br>ISSN: 1439-4235, DOI: 10.1002/cphc.201601204<br>* page 551; figure 2; compounds BFA-1T *<br>* BHA-, BpMA-, BMOA-1T;<br>figure 1 *<br>* figure 7, compound BMOA-4T *<br>* page 558, synthesis of BHA-3T *<br>----- | 1-3,5-9 | TECHNICAL FIELDS SEARCHED (IPC)<br>C07D<br>H01L |
| X | YING SUN ET AL: "Chemically Doped and Cross-linked Hole-Transporting Materials as an Efficient Anode Buffer Layer for Polymer Solar Cells",<br>CHEMISTRY OF MATERIALS,<br>vol. 23, no. 22,<br>22 November 2011 (2011-11-22), pages 5006-5015, XP055121934,<br>ISSN: 0897-4756, DOI: 10.1021/cm2024235<br>* scheme 3, compound T-BTPA, intermediates 2c, 3c, 4c *<br>-----<br>-/-- | 1-3,5-9 | |

1

~~The present search report has been drawn up for all claims~~

| Place of search | Date of completion of the search | Examiner |
|---|---|---|
| Munich | 18 March 2021 | Gutke, Hans-Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 21 0564

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHANG F ET AL: "Triphenylamine-based dyes for dye-sensitized solar cells", DYES AND PIGMENTS, ELSEVIER APPLIED SCIENCE PUBLISHERS BARKING, GB, vol. 81, no. 3, 1 June 2009 (2009-06-01), pages 224-230, XP025879861, ISSN: 0143-7208, DOI: 10.1016/J.DYEPIG.2008.10.012 [retrieved on 2008-11-06] * figure 2; compound 7 * ----- | 1-3,5-9 | |
| | | | TECHNICAL FIELDS SEARCHED (IPC) |

1

~~The present search report has been drawn up for all claims~~

| Place of search | Date of completion of the search | Examiner |
|---|---|---|
| Munich | 18 March 2021 | Gutke, Hans-Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 20 21 0564

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-3, 5-9, 13-23(all partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

**LACK OF UNITY OF INVENTION SHEET B**

Application Number
EP 20 21 0564

The Search Division considers that the present European patent application does not comply with the requirements of unity of invention and relates to several inventions or groups of inventions, namely:

1. claims: 1-3, 5-9, 13-23(all partially)

   compounds of formula 1 wherein n is 1

   ---

2. claims: 1-10, 12-23(all partially)

   compounds of formula 1 wherein n is 2

   ---

3. claims: 1-10, 12-23(all partially)

   compounds of formula 1 wherein n is 3

   ---

4. claims: 11(completely); 1-9, 12-23(partially)

   compounds of formula 1 wherein n is 4

   ---

5. claims: 1-3, 5-9, 13-23(all partially)

   compounds of formula 1 wherein n is more than 4

   ---

## ANNEX TO THE EUROPEAN SEARCH REPORT ON EUROPEAN PATENT APPLICATION NO. EP 20 21 0564

This annex lists the patent family members relating to the patent documents cited in the above-mentioned European search report.
The members are as contained in the European Patent Office EDP file on
The European Patent Office is in no way liable for these particulars which are merely given for the purpose of information.

18-03-2021

| Patent document cited in search report | Publication date | Patent family member(s) | Publication date |
|---|---|---|---|
| WO 2015084256 A1 | 11-06-2015 | CN 104485424 A | 01-04-2015 |
| | | TW 201528530 A | 16-07-2015 |
| | | WO 2015084256 A1 | 11-06-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015114521 A1
- WO 2015161989 A1

### Non-patent literature cited in the description


- **H.-S. KIM ; C.-R. LEE ; J.-H. IM ; K.-B. LEE ; T. MOEHL ; A. MARCHIORO ; S.-J. MOON ; R. HUMPHRY-BAKER ; J.-H. YUM ; J. E. MOSER.** *Sci. 2012, Rep,* vol. 2, 591
- **Y. CHEN.** *Adv. Energy Mater.,* 2019, vol. 9, 1901268
- **S. VEGIRAJU.** *Adv. Func. Mater.,* 2019, 1905393
- **I. ZIMMERMANN.** *Angew. Chem. Int. Ed,* 2019, vol. 58, 1072-1076
- **D. BI.** *Sci. Adv.,* 2016, vol. 2, 1501170
- **H. LI et al.** *ChemSusChem,* 2014, vol. 7, 3420-3425
- **THIRUMAL et al.** *J. Mater. Chem. A,* 2014, vol. 2, 6305-6309
- **MONA SHASTI et al.** *Org. Lett.,* 2019, vol. 21, 3261-3264
- **LIANG DUAN.** *ACS Appl. Energy Mater,* 2020, vol. 3, 1672-1683
- *Macromolecules,* 1998, vol. 31, 4838-4844
- *Adv. Funct Mater,* 2007, vol. 17, 1163-1171
- *J. Am. Chem. Soc.,* 2004, vol. 126, 13480-13501